# EUROPÄISCHE PATENTANMELDUNG

(11) **EP 2 431 334 A1**
(43) Veröffentlichungstag der Anmeldung: **21.03.2012**
(21) Anmeldenummer: 10009700.5
(22) Anmeldetag: 16.09.2010
(51) Int. Cl.: C02F 1/42, C02F 101/22, C02F 103/16, C02F 101/14, C02F 101/34, C02F 101/36, C02F 101/30, C02F 1/28, C02F 101/20

(54) **Behandlung von Abwässern aus der Galvanikindustrie**

(71) Anmelder: LANXESS Deutschland GmbH, 51369 Leverkusen (DE)
(72) Erfinder: Podesta, Wolfgang Dr., 51381 Leverkusen (DE); Neumann, Stephan Dr., 51375 Leverkusen (DE)

(57) **Zusammenfassung**

Die Erfindung betrifft ein Verfahren zur Abtrennung von fluorierten Säuren, insbesondere Perfluorcarbonsäuren wie perfluoroctancarbonsäure (PFOA) und Perfluorsulfonsäuren wie Perfluoroctansulfonsäure (PFOS) oder deren Salzen aus metallhaltigen, wässrigen Lösungen wie insbesondere solchen, die in der Galvanikindustrie auftreten, mit Hilfe von Anionentauschern.

## Beschreibung

Die Erfindung betrifft ein Verfahren zur Abtrennung von fluorierten Säuren, insbesondere Perfluorcarbonsäuren und Perfluorsulfonsäuren oder deren Salzen aus metallhaltigen, wässrigen Lösungen wie insbesondere solchen, die in der Galvanikindustrie auftreten, mit Hilfe von Anionentauschern.

Fluorierte Säuren und deren Salze, wie insbesondere Perfluorsulfonsäuren (PFOS) und deren Alkalimetall- und Ammoniumsalze, finden häufig Verwendung als Netzmittel bzw. Sprühnebelunterdrücker in der Galvanikindustrie, wie zum Beispiel in der Hart- und Dekoratiwerchromung.

Der wesentliche Nutzen besteht hierbei in der hohen Stabilität der fluorierten Säuren, der gleichmäßigen Benetzung der zu verchromenden Teile sowie der Vermeidung von giftigen Chrom(VI)-Austrägen aus den eingesetzten Chromschwefelsäure- bzw. Verchromungsbädern und die damit verbundenen Belastung von Mensch und Umwelt.

Nachteilig ist jedoch die Persistenz sowie die schlechte biologische Abbaubarkeit sowie die damit verbundene Anreicherung in der Nahrungskette. Aus diesem Grund gibt es in vielen Ländern Bestrebungen, die Einträge in die Umwelt unter gesetzliche Auflagen zu stellen, in der Europäischen Union bespielsweise durch die EU-Direktive 2006/122/EG.

Aus diesem Grund wurde gerade in den letzten Jahren intensiv nach einer Methode zur möglichst quantitativen Entfernung von fluorierten Säuren aus Abwässern galvanischer Betriebe gesucht.

Dabei wurden unterschiedliche Ansätze wie beispielsweise die Absorption an Aktivkohle bzw. gefälltem Calciumfluorid, die Abtrennung durch Verdampfung von Wasser und Flüssig-Flüssig-Extraktion angewandt.

In DE 199 53 285 A der Einsatz von Anionenaustauschern zur Entfernung von Perfluoroctansäure enthaltenden Abwässern aus der Herstellung von Fluorpolymeren beschrieben, wobei die auf dem Harz absorbierte Perfluoroctansäure mittels ammoniakhaltiger, wasserlöslicher organischer Lösemittel mit einem Siedepunkt <150°C eluiert werden kann.

Aus DE-A 2 044 986 ist ein Verfahren zur Gewinnung von Perfluorcarbonsäuren aus verdünnten wässrigen Lösungen bekannt, wobei letztere in Kontakt mit einem schwach basischen Anionenaustauscherharz gebracht wird und die darauf absorbierte Perfluorcarbonsäure mittels einer wässrigen Ammoniaklösung eluiert wird.

Aus US 5,442,097 ist ein Verfahren zur Rückgewinnung fluorierter Carbonsäuren aus Emulsionsplolymerisaten bekannt. Die hierbei als Tenside eingesetzten fluorhaltigen Carbonsäuren werden mittels eines geeigneten Alkohols in den entsprechenden Ester umgesetzt und letzterer destillativ abgetrennt.

In JP 2010158662 wird ein Verfahren zur Entfernung von fluorierten Tensiden wie z.B. Perfluoroctansäure aus wässrigen Lösungen mittels Akltivkohlefiltern beschrieben, das gemäß JP 2010029763 dadurch modifiziert wird, dass vor dem Aktivkohlefilter zusätzlich eine Behandlung der Abwässer mit Nanobläschen erfolgt, die zumindest teilweise zu einer Zerstörung der persistenten Perfluorverbindungen führt.

In WO 2008/101137 wird schließlich ein Verfahren zur Entfernung von Fluorchemikalien einschließlich Perfluoroetansulfonaten bzw. -carboxylaten aus Grund- und Oberflächenwässern mittels Ionenaustauschern beschrieben. Hierbei kann die Konzentration der Fluorchemikalien im ppm- bis ppb-Bereich liegen.

All diesen Verfahren ist gemeinsam, dass sie eine unzureichende Selektivität der eingesetzten Agenzien bzgl. der abzutrennenden fluorierten Säuren aufweisen oder große Mengen an Prozesschemikalien wie Ammoniak, Alkohole oder andere organische Lösemittel zur Regeneration der freien Säuren bzw. Destillation der Ester benötigen. Dabei wird der zunächst erzielte Anreicherungseffekt überwiegend wieder aufgehoben.

Auch wurden die Verfahren teilweise nur für die Entfernung von fluorierten Carbonsäuren entwickelt, eine Übertragbarkeit auf die entsprechenden Sulfonsäuren ist aber wegen der unterschiedlichen physikochemischen Eigenschaften nicht ohne Weiteres möglich.

Darüber hinaus muss im Falle der Abwässeraufbereitung aus galvanischen Verchromungsanlagen der teilweise sehr hohe Gehalt an Begleitbestandteilen wie beispielsweise Schwefelsäure, Chromsäure sowie diverse Metallionen berücksichtigt werden.

Auch können die in den eingesetzten Perfluoroctansulfonsäuren herstellungsbedingt ebenfalls enthaltenen kürzerkettigen Verbindungen wie beispielsweise Perfluorbutansulfonsäure bzw. deren Salze aufgrund der geringeren Bindung an die eingesetzten Agenzien typischerweise nur in deutlich geringerem Umfang aus der wässrigen Phase abgetrennt werden, was insbesondere auf Aktivkohlefilter zutrifft.

Es bestand daher die Aufgabe, ein Verfahren bereitzustellen, das die möglichst vollständige Abtrennung von fluorierten Säuren aus metallhaltigen, wässrigen Lösungen erlaubt.

Es wurde nun ein Verfahren zur Abtrennung von fluorierten Säuren oder deren Salze aus ihren metallhaltigen, wässrigen Lösungen gefunden, das dadurch gekennzeichnet ist, dass vorgenannte Lösungen mit zumindest einem schwach oder stark basischen Anionentauscher kontaktiert werden.

Der Rahmen der Erfindung umfasst die vor- und nachstehend aufgeführten, allgemeinen oder in Vorzugsbereichen aufgeführten Restedefinitionen bzw. Parameter auch in beliebiger Kombination untereinander.

Der Begriff "wässrige Lösung" bedeutet im Rahmen der Erfindung ein flüssiges Medium mit einem Feststoffanteil von weniger als 5 Gew.-% bevorzugt weniger als 1 Gew.-% und besonders bevorzugt weniger als 0,05 Gew.-%, das zumindest 80 Gew.-%, bevorzugt zumindest 90 Gew.-% Wasser sowie zumindest eine fluorierte Säure oder zumindest ein Salz einer fluorierten Säure enthält, wobei die Gesamtmenge an fluorierter Säure oder Salzen fluorierter Säure 1 bis 200.000 µg/l vorzugsweise 10 bis 2.000 µg/l, besonders bevorzugt 10 bis 1.000 µg/l und ganz besonders bevorzugt 10 bis 500 µg/l beträgt.

Fluorierte Säuren im Sinne der Erfindung sind solche, die 1 bis 10 Kohlenstoffatome und zumindest ein Fluoratom aufweisen und bei Standardbedingungen einen pKs von 6,0 oder weniger aufweisen. Dabei können ein oder mehrere, bevorzugt eine Säuregruppe vorhanden sein, wobei sich die Angabe des pKs-Wertes bei mehrwertigen Säuren auf die jeweils erste Deprotonierungsstufe bezieht.

Bevorzugte fluorierte Säuren im Sinne der Erfindung sind Poly- und Perfluorcarbonsäuren der Formel (I) und Poly- und Perfluorsulfonsäuren der Formel (II)

F-(CF₂)ₙ-(CH₂)ₘ-COOH (I)

F-(CF₂)ₙ-(CH₂)ₘ-SO₂OH (II)

in denen jeweils
n für eine ganze Zahl von 3 bis 10, bevorzugt 4 bis 8 und
m für 0 oder eine ganze Zahl von 1 bis 4 steht.

Ganz besonders bevorzugte fluorierte Säuren sind Perfluoroctansulfonsäure, Perfluoroctancarbonsäure, Perfluorbutansulfonsäure und Perfluorbutancarbonsäure.

Die für die fluorierten Säuren vorstehend genannten Definitionen Bereiche und Vorzugsbereiche gelten für die entsprechenden Salze von fluorierten Säuren völlig analog. Unter einem Salz einer fluorierten Säure ist eine Verbindung zu verstehen in der das Säureproton durch ein anderes Kation, wie zum Beispiel ein Metall-Kation oder Ammonium-Ion, wie beispielsweise ein organisches primäres, sekundäres, tertiäres oder quaternäres Ammonium-Ion wie beispielsweise einem Tetraethylammoniumion ersetzt ist.

Metallhaltig im Sinne der Erfindung bedeutet einen Gehalt an zumindest einer Übergangsmetallverbindung, wobei der Gehalt an Übergangsmetallverbindungen berechnet auf das jeweilige Übergangsmetallsmetalloxid mit der gleichen Oxidationsstufe wie die zumindest eine enthaltene Übergangsmetallverbindung 10 mg/l bis 100 g/l, vorzugsweise von 50 mg/l bis 10 g/l und besonders bevorzugt von 100 mg/l bis 5 g/l beträgt.

Vorzugsweise sind die verdünnten, metallhaltigen wässrigen Lösungen chromhaltig und weisen einen Chromgehalt von 10 mg/l bis 100 g/l, vorzugsweise von 50 mg/l bis 10 g/l und besonders bevorzugt von 100 mg/l bis 5 g/l berechnet auf Chrom(VI)oxid auf, wobei das Chrom in Form von Chromsäure bzw. Chromaten und oder alternativ in Form von Dichromsäure bzw. Dichromaten vorliegt.

Die chromhaltigen Lösungen können auch weiterhin kupfer-, eisen- oder nickelhaltig sein.

In einer beispielhaften Ausführungsform beträgt der pH-Wert der metallhaltigen, wässrigen Lösungen von fluorierten Säuren oder deren Salze von 0 bis 7 bei Standardbedingungen, bevorzugt von 0,0 bis 5,5 und besonders bevorzugt 1,0 bis 5,5.

In einer anderen Ausführungsform beträgt der pH-Wert 0,0 bis 3,0 bevorzugt 1,0 bis 3,0 bei Standardbedingungen.

Weisen die verdünnten, metallhaltigen wässrigen Lösungen einen pH-Wert von weniger als 7 bei Standardbedingungen auf, enthalten sie vorzugsweise Schwefelsäure und/oder Hydrogensulfat.

Die erfindungsgemäß eingesetzten metallhaltigen, wässrigen Lösungen sind bevorzugt Abwässer aus Galvanikanlagen wie insbesondere Abwässer aus der Hart- oder Dekoratiwerchromung, einschließlich der Abwässer der typischerweise den Galvanikanlagen vorgeschalteten Beizbäder sowie der Abwässer aus den Spülkaskaden und Abgaswäschern von Galvanikanlagen anfallenden Abwässer.

Die Abwässer können beispielsweise entweder direkt nach dem Galvanisierungs- bzw. Verchromungsprozess gesammelt und dem erfindungsgemäßen Verfahren zugeführt werden, oder aber nach der Reduzierung der noch enthaltenen Übergangsmetallverbindungen, wie insbesondere Chrom(VI)-Verbindungen, sowie gegebenenfalls nach Abtrennung von Niederschlägen beispielsweise mittels Kammerfilterpressen.

Geeignete Anionentauscher umfassen stark basische und schwach basische Aniontauscher, wobei unter stark basischen Aniontauschern insbesondere solche zu verstehen sind, die quaternäre Ammonium-Ionen enthalten und unter schwach basischen solche, die als Strukturelement primäre, sekundäre oder tertiäre Amingruppen oder deren korrespondierende Ammoniumionen enthalten.

Bevorzugte stark basische Aniontauscher sind solche, die das Strukturelement der Formel (III) aufweisen

-N⁺(R¹R²R³)X⁻ (III)

worin
R¹, R² und R³ jeweils unabhängig voneinander für C₁-C₁₂-Alkyl stehen, das entweder nicht, einfach oder mehrfach durch Hydroxy- oder C₁-C₄-Alkoxyweiter substituiert sein kann, oder zwei der Reste zusammen für C₂-C₁₂-Alkylen stehen, das einfach oder mehrfach durch Hydroxy- oder C₁-C₄-Alkoxy substituiert sein kann und
X⁻ für ein Anion steht, das in einer bevorzugten Verfahrensform aus der Gruppe Fluorid, Chlorid, Bromid, Hydroxid, Nitrat, Hydrogensulfat und Sulfat ausgewählt ist

Bevorzugte schwach basische Aniontauscher sind solche, die das Strukturelement der Formel (IV) oder das Strukturelement der Formel (V) aufweisen oder Strukturelemente der Formeln (IV) und (V) aufweisen

-N⁺(R⁴R⁵R⁶)X (IV)

worin
R⁴, R⁵ und R⁶ jeweils unabhängig voneinander für Wasserstoff oder C₁-C₁₂-Alkyl stehen, das entweder nicht, einfach oder mehrfach durch Hydroxy- oder C₁-C₄-Alkoxyweiter substituiert sein kann, oder, sofern zwei der Reste R⁴, R⁵ und R⁶ nicht für Wasserstoff stehen diese Reste zusammen für C₂-C₁₂-Alkylen stehen, das einfach oder mehrfach durch Hydroxy- oder C₁-C₄-Alkoxy substituiert sein kann und wobei jedoch zumindest einer, bevorzugt ein oder zwei, besonders bevorzugt einer der Reste R⁴, R⁵ und R⁶ für Wasserstoff steht und
X- für ein Anion steht, das in einer bevorzugten Verfahrensform aus der Gruppe Fluorid, Chlorid, Bromid, Hydroxid, Nitrat, Hydrogensulfat und Sulfat, vorzugsweise Hydrogensulfat und Sulfat ausgewählt ist

-N(R⁷R⁸) (V)

worin
R⁷ und R⁸ jeweils unabhängig voneinander für Wasserstoff oder C₁-C₁₂-Alkyl stehen, das entweder nicht, einfach oder mehrfach durch Hydroxy- oder C₁-C₄-Alkoxyweiter substituiert sein kann, oder, sofern zwei der Reste R⁷ und R⁸ nicht für Wasserstoff stehen diese Reste zusammen für C₂-C₁₂-Alkylen stehen, das einfach oder mehrfach durch Hydroxy- oder C₁-C₄-Alkoxy substituiert sein kann.

Prinzipiell umfassen geeignete Ionentauscher auch solche, die die Strukturelemente der Formeln (III) sowie (IV) und/oder (V) aufweisen.

Erfindungsgemäß werden die verdünnten, metallhaltigen wässrigen Lösungen mit zumindest einem schwach oder stark basischen Anionentauscher kontaktiert.

Das schließt sowohl die Kontaktierung mit mehreren schwach basischen oder mehreren stark basischen Anionentauschern als auch die Kontaktierung mit zumindest einem schwach basischen und zumindest einem stark basischen Anionentauscher mit ein.

Als schwach basische Ionentauscher sind bevorzugt solche, die das Strukturelement der Formeln (IV) und/oder (V) aufweisen.

Besonders bevorzugte Anionentauscher sind Lewatit® MP 62, ein schwach basischer, makroporöser Aniontauscher mit tertiären Aminogruppen, Lewatit ® MP 64 ein schwach basischer, makroporöser Aniontauscher auf Basis eines Styrol-Divinylbenzol-Copolymers, Lewatit ® Monoplus MP 500 ein stark basischer, makroporöser Aniontauscher und Lewatit ® Monoplus MP 600 ein stark basischer, makroporöser Aniontauscher, alle von der Firma Lanxess Deutschland GmbH.

Weiterhin seien als geeignete Anionentauscher die kommerziell verfügbaren anionenaustauschenden Amberlite® oder Duolite® der Firma Dow Chemical genannt. Vorzugsweise liegt der Anionentauscher zumindest teilweise in Sulfat- oder Hydrogensulfat-Form vor, d.h. an die Anionentauscher sind über ionische Wechselwirkungen Sulfat- oder Hydrogensulfat-Anionen gebunden.

Typischerweise wird das erreicht, indem der Aniontauscher mit verdünnter, wässriger Schwefelsäure, die typischerweise eine Konzentration von 0,1 bis 20 Gew.-% berechnet auf H₂SO₄ aufweist, in Kontakt gebracht wird.

Das Inkontaktbringen der metallhaltigen, wässrigen Lösungen von fluorierten Säuren oder ihrer Salze mit dem Anionentauscher kann in an sich bekannter Weise erfolgen, wobei beispielsweise die Anionentauscher in üblichen Apparaturen wie Röhren oder Säulen angeordnet sein können, die von den verdünnten, metallhaltigen wässrigen Lösungen durchströmt werden.

Das Inkontaktbringen der metallhaltigen, wässrigen Lösungen von fluorierten Säuren oder ihrer Salze mit dem Anionentauscher erfolgt beispielsweise mit einer Geschwindigkeit von 0,5 bis 200, bevorzugt 2 bis 100 Volumenteilen metallhaltiger, wässriger Lösung pro Stunde und Volumenteil Anionentauscher..

Das nach dem Inkontaktbringen der metallhaltigen, wässrigen Lösungen von fluorierten Säuren oder ihrer Salze mit dem Anionentauscher verbleibende Abwasser weist typischerweise einen deutlich niedrigeren Gehalt an fluorierten Säuren oder ihren Salzen auf als vor dem Inkontaktbringen, wobei der Prozess vorzugsweise so gesteuert wird, dass zumindest 90 Gew.-% der in den eingesetzten, verdünnten wässrigen Lösungen vorhandenen fluorierten Säuren oder ihre Salze durch den Anionentauscher gebunden werden, vorzugsweise 95 Gew.-%.

Beispielsweise ist dies gewährleistet, wenn der Anionentauscher nach Durchströmen von oder Inkontaktbringen mit einer gewissen Menge von verdünnter, metallhaltiger wässriger Lösung regeneriert oder ersetzt wird.

Die Kapazität des Aniontauschers für fluorierte Säuren oder deren Salze hängt unter anderem vom gewählten Typ des Anionentauschers und Art und Gehalt der eingesetzten verdünnten, metallhaltigen wässrigen Lösungen an fluorierten Säuren oder deren Salze sowie weiteren Anionen wie im Falle von Abwässern aus der Verchromung beispielsweise Chromaten. Diese kann jedoch in einfachen Vorversuchen von Fachmann in an sich bekannter Weise ermittelt werden

Gegebenenfalls kann das Abwasser zur Entfernung möglicherweise restlicher Anteile an fluorierten Säuren oder ihren Salzen mit üblichen Adsorbenzien wie zum Beispiel Aktivkohle in Kontakt gebracht werden.

Auch ist es möglich, Abwässer, die zunächst mit üblichen Absorbenzien wie zum Beispiel Aktivkohle zwecks Reduzierung des Gehaltes an fluorierten Säuren oder anderen Verunreinigungen in Kontakt gebracht wurden, anschließend mittels Anionenaustauschern auf einen noch niedrigeren Gehalt an fluorierten Säuren gebracht werden.

Diese Variante ist besonders dann einzusetzen, wenn ein Aktivkohlefilter bereits vorhanden ist, der gewünschte Gehalt an fluorierten Säuren aber nicht erreicht werden kann. Dies gilt besonders für Abwässer, die neben Perfluoroctansulfonsäure bzw. -carbonsäure noch kürzerkettige Perfluorsulfonsäuren bzw. -carbonsäuren enthalten, da diese typischerweise in geringerem Maße an Aktivkohle absorbiert werden.

Die über ionische Bindungen an die Anionenaustauscher gebundenen fluorierten Säuren bzw. deren Anionen sind aufgrund der starken Wechselwirkung mit dem Anionenaustauscher im wässrigen Milieu, zum Beispiel mit verdünnter Natronlauge, in der Regel nur schwer eluierbar.

Es ist daher bevorzugt, die mit fluorierten Säuren beladenen Anionenaustauscher bei Temperaturen oberhalb 1100°C zu verbrennen. Dadurch ist sichergestellt, dass die fluorierten Säuren quantitativ beseitigt und damit die Umwelt von einer möglichen Exposition befreit wird.

Der Vorteil der Erfindung liegt in der überlegenen Abtrennung von fluorierten Säuren oder deren Salzen aus verdünnten, metallhaltigen wässrigen Lösungen im Vergleich zum Stand der Technik.

### Beispiele

### Beispiele 1 bis 4

500 ml eines stark sauren Spülwassers mit einem pH-Wert von 1,5 aus der Dekorativverchromung, welches noch 0,75 g/l Chromat berechnet als CrO₃ und ca. 0,5 mg/l an fluorierten Säuren enthielt, wurden mit einer Geschwindigkeit von1 l/h über 100 ml des in Tabelle 1 angegebenen Materials in einer Chromatographiesäule gepumpt. Das Filtrat wurde anschließend homogenisiert und auf fluorierte Säuren untersucht. Tabelle 1 gibt die Ergebnisse wieder.

**Tabelle 1: Adsorption an verschiedenen Medien**

| Beispiel | Probe/Material Ausgangsprobe | PFBA [µg/l] < 3 | PFBS [µg/l] 64 | PFOA [µg/l] < 3 | PFOS [µg/l] 411 |
|---|---|---|---|---|---|
| 1 (zum Vergleich) | OC 1064 | 1,4 | 7,6 | < 0,3 | 44 |
| 2 (zum Vergleich) | Lewatit®AF5 | 1,5 | 7,8 | < 0,3 | 44 |
| 3 | Lewatit® MP62 | 1,4 | < 0,3 | < 0,3 | < 0,3 |
| 4 | Lewatit®MP500 | 0,8 | < 0,3 | < 0,3 | < 0,3 |

PFBA = Perfluorbutancarbonsäure
PFBS = Perfluorbutansulfonsäure
PFOA = Perfluoroctancarbonsäure
PFOS = Perfluoroctansulfonsäure
OC1064 = Adsorberharz

Lewatit®AFS = Aktivkohle

Lewatit®MP62 = schwach basischer Anionenaustauscher (Lanxess Deutschland GmbH) Lewatit® Monoplus M500 = stark basischer Anionenaustauscher (Lanxess Deutschland GmbH)

Aus der Tabelle ist ersichtlich, dass die Ausgangskonzentration an fluorierten Säuren im Eluat besonders im Fall der Aniontauscher besonders stark reduziert wird.

Dabei wird im Gegensatz zum Einsatz des Adsorberharzes OC 1064 - neben der Hauptkomponente (PFOS) auch die Nebenkomponente (PFBS) vollständig absorbiert.

Erfindungsgemäß werden über 99,5 bzw. 99,9 Gew.-% des in der Ausgangsprobe enthaltenen PFBS bzw. PFOS entfernt, ohne dass es dabei einer weiteren Probenvorbereitung bedurfte.

Auch stört die im Abwasser enthaltene Chromsäure das Ergebnis überraschenderweise nicht.

### Beispiel 5

Die Spülwässer einer Dekoratiwerchromung mit einem pH-Wert von etwa 1,5 aus der Dekoratiwerchromung, welches im Durchschnitt noch 0,75 g/l Chromat berechnet als CrO₃ aufwies, wurden direkt über Aktivkohle-Filterkerzen und anschließend über zwei mit Lewatit®MP62 befüllte Kartuschen von je 50 l Harzvolumen geführt. Am Auslauf der Ionenaustauscher-Kartuschen wurden zweimal wöchentlich Proben gezogen und diese auf fluorierte Säuren analysiert. Der Spülwässerdurchsatz betrug 120 m³ pro Tag.

Tabelle 2 zeigt die Ergebnisse:

**Tabelle 2:**

| Beispiel | Probe Ausgangsprobe | PFBS [µg/l] 3 | PFOS [µg/l] 63 |
|---|---|---|---|
| 5a | Eluat nach 1. Tag | < 0,3 | 0,6 |
| 5b | Eluat nach 4. Tag | < 0,3 | 0,6 |
| 5c | Eluat nach 7. Tag | < 0,3 | 1,5 |

Die Ergebnisse zeigen, dass die im Abwasser enthaltene Perfluorbutansulfonsäure (PFBS) zu mehr als 90 %, Perfluoroctansulfonsäure (PFOS) nahezu quantitativ entfernt wurde.

## Patentansprüche

1. Verfahren zur Abtrennung von fluorierten Säuren oder deren Salze aus ihren metallhaltigen, wässrigen Lösungen, **dadurch gekennzeichnet, dass** vorgenannte Lösungen mit zumindest einem schwach oder stark basischen Anionentauscher kontaktiert werden.

2. Verfahren nach Anspruch 1, **dadurch gekennzeichnet, dass** die metallhaltigen, wässrigen Lösungen eine Gesamtmenge an fluorierten Säuren oder Salzen fluorierter Säuren von 1 bis 200.000 µg/l enthalten.

3. Verfahren nach Anspruch 1 oder 2, **dadurch gekennzeichnet, dass** die fluorierten Säuren folgende sind:
Poly- und Perfluorcarbonsäuren der Formel (I) bzw. Poly- und Perfluorsulfonsäuren der Formel (II)
F-(CF₂)ₙ-(CH₂)ₘ-COOH (I)
F-(CF₂)ₙ-(CH₂)ₘ-SO₂OH (II)
in denen jeweils
n für eine ganze Zahl von 3 bis 10, bevorzugt 4 bis 8 und
m für 0 oder eine ganze Zahl von 1 bis 4 steht.

4. Verfahren nach einem der Ansprüche 1 bis 3, **dadurch gekennzeichnet, dass** die metallhaltigen, wässrigen Lösungen zumindest eine Übergangsmetallverbindung enthalten, wobei der Gehalt an Übergangsmetallverbindungen berechnet auf das jeweilige Übergangsmetallsmetalloxid mit der gleichen Oxidationsstufe wie die zumindest eine enthaltene Übergangsmetallverbindung, 10 mg/l bis 100 g/l beträgt.

5. Verfahren nach einem der Ansprüche 1 bis 4, **dadurch gekennzeichnet, dass** die metallhaltigen, wässrigen Lösungen chromhaltig sind und weisen einen Chromgehalt von 10 mg/l bis 100 g/l aufweisen.

6. Verfahren nach einem der Ansprüche 1 bis 5, **dadurch gekennzeichnet, dass** der pH-Wert der metallhaltigen, wässrigen Lösungen von fluorierten Säuren oder deren Salze von 0 bis 7 bei Standardbedingungen beträgt.

7. Verfahren nach einem der Ansprüche 1 bis 6, **dadurch gekennzeichnet, dass** der pH-Wert der metallhaltigen, wässrigen Lösungen von fluorierten Säuren oder deren Salze von 0,0 bis 3,0 bei Standardbedingungen beträgt.

8. Verfahren nach einem der Ansprüche 6 oder 7, **dadurch gekennzeichnet, dass** für den Fall, dass die metallhaltigen wässrigen Lösungen einen pH-Wert von weniger als 7 bei Standardbedingungen aufweisen, die Lösungen weiterhin Schwefelsäure und/oder Hydrogensulfat enthalten.

9. Verfahren nach einem der Ansprüche 1 bis 8, **dadurch gekennzeichnet, dass** metallhaltigen, wässrigen Lösungen Abwässer aus Galvanikanlagen sind.

10. Verfahren nach einem der Ansprüche 1 bis 10, **dadurch gekennzeichnet, dass** als Aniontauscher solche eingesetzt werden die das Strukturelement der Formel (IV) oder das Strukturelement der Formel (V) aufweisen oder Strukturelemente der Formeln (IV) und (V) aufweisen
-N⁺(R⁴R⁵R⁶)X⁻ (IV)
worin
R⁴, R⁵ und R⁶ jeweils unabhängig voneinander für Wasserstoff oder C₁-C₁₂-Alkyl
stehen, das entweder nicht, einfach oder mehrfach durch Hydroxy-oder C₁-C₄-Alkoxy weiter substituiert sein kann, oder, sofern zwei der Reste R⁴, R⁵ und R⁶ nicht für Wasserstoff stehen diese Reste zusammen für C₂-C₁₂-Alkylen stehen, das nicht, einfach oder mehrfach durch Hydroxy- oder C₁-C₄-Alkoxy substituiert sein kann und
Wobei jedoch zumindest einer der Reste R⁴, R⁵ und R⁶ für Wasserstoff steht und
X- für ein Anion steht
-N(R⁷R⁸) (V)
worin
R⁷ und R⁸ jeweils unabhängig voneinander für Wasserstoff oder C₁-C₁₂-Alkyl
stehen, das entweder nicht, einfach oder mehrfach durch Hydroxy-oder C₁-C₄-Alkoxyweiter substituiert sein kann, oder, sofern zwei der Reste R⁷ und R⁸ nicht für Wasserstoff stehen diese Reste zusammen für C₂-C₁₂-Alkylen stehen, das nicht, einfach oder mehrfach durch Hydroxy- oder C₁-C₄-Alkoxy substituiert sein kann.

11. Verfahren nach einem der Ansprüche 1 bis 10, **dadurch gekennzeichnet, dass** das Inkontaktbringen der metallhaltigen, wässrigen Lösungen mit einer Geschwindigkeit von 0,5 bis 200 Volumenteilen metallhaltiger, wässriger Lösung pro Stunde und Volumenteil Anionentauscher erfolgt.

12. Verfahren nach einem der Ansprüche 1 bis 11, **dadurch gekennzeichnet, dass** die metallhaltigen, wässrigen Lösungen nach dem Inkontaktbringen mit dem Anionentauscher zur Entfernung möglicherweise restlicher Anteile an fluorierten Säuren oder ihren Salzen mit Adsorbenzien in Kontakt gebracht werden.

13. Verfahren nach einem der Ansprüche 1 bis 12, **dadurch gekennzeichnet, dass** die metallhaltigen, wässrigen Lösungen vor dem Inkontaktbringen mit dem Anionentauscher zur Entfernung von Anteilen an fluorierten Säuren oder ihren Salzen mit Adsorbenzien in Kontakt gebracht werden.

14. Verfahren nach einem der Ansprüche 1 bis 13, **dadurch gekennzeichnet, dass** die nach Durchführung des Verfahrens mit fluorierten Säuren beladenen Anionenaustauscher bei Temperaturen oberhalb von 1100°C verbrannt werden.

15. Verwendung von Anionentauschern in einem Verfahren nach einem der Ansprüche 1 bis 14.
